# EUROPEAN PATENT SPECIFICATION

(11) **EP 0 462 412 B1**
(45) Date of publication and mention of the grant of the patent: **10.07.1996**
(21) Application number: 91108316.0
(22) Date of filing: 23.05.1991
(51) Int. Cl.: A61M 16/10, A61M 16/08, G01N 1/22, A61B 5/097

(54) **Y-piece connector for ventilator**
Y-förmiges Verbindungsstück für Beatmungsgerät
Connecteur en forme de pièce Y pour ventilateur artificiel

(30) Priority: 18.06.1990 SE 9002162; 02.11.1990 SE 9003505
(43) Date of publication of application: 27.12.1991
(60) Divisional application: 94102959.7
(73) Proprietor: ENGSTRÖM MEDICAL AB, S-161 02 Bromma (SE)
(72) Inventor: Kihlberg, Ake, S-183 50 Täby (SE); Tryggvason, Ragnar, S-240 21 Löddeköpinge (SE); Wikefeldt, Per, S-175 45 Järfälla (SE)
(74) Representative: Lettström, Richard Wilhelm

(56) References cited:
- EP-A- 0 265 163
- WO-A-89/04684
- WO-A-90/04425
- FR-A- 1 121 482
- FR-A- 2 315 955
- GB-A- 2 116 434
- US-A- 4 456 014
- US-A- 4 558 708

## Description

The present invention relates to a connection arrangement for connecting a patient to a respirator, anaesthesia machine or similar, comprising a patient attachment piece, a so-called Y-piece disconnectable from said patient attachment piece and arranged to join said patient attachment piece to an inhalation tube and an exhalation tube, respectively, a bacteria filter and a heat and moisture exchange system.

EP-A-0 265 163 discloses a connection arrangement according to the pre-characterizing portion of claim 1 and comprises a combined bacteria filter and moisture and heat exchanger in a house to which a Y-piece and a patient attachment piece can be connected.

From the following description it will be evident that the expression "Y-piece" also includes shapes other than a conventional "Y". Of importance is only that said patient attachment piece is able to be connected to an inhalation resp an exhalation tube. The expression "Y-piece" also includes for example T-connections and even connections for connecting coaxially arranged inhalation and exhalation tubes.

The patient attachment piece can by way of example be designed in accordance with US-A-4 516 573. In this patent there is described a bellowed patient attachment piece which contains a wad of moisture and heat absorbing material. In this way heat and moisture is taken up from the exhaled gas and given to the inhaled gas. Such patient attachment pieces are often connected with various types of Y-pieces and are possibly also equipped with various types of bacteria filters. The more components which are connected leads to a bulkier construction which can be inconvenient for the patient. Furthermore, the connection of a plurality of components leads to the risk of, on the one hand, leakage and, on the other hand, incorrectly connected components.

The bulkier construction leads to the problem that the so called dead volume for the breathing gas increases when a plurality of components are combined.

With the attachment of a patient to a respirator, anaesthesia machine or similar, the need arises for a continuous or intermittent sampling of the exhaled gas respectively a proximal pressure measuring. This should take place as close to the patient as possible and can occur for example in the way described in US-A-4 838 258, i.e. with the help of a tube which extends from the respirator itself through the exhalation tube to a point near the patient. The disadvantage with the constructions described in this patent is, however, that the withdrawn sample can be, on the one hand, very moist, and, on the other hand, contaminated with bacteria.

The above-mentioned problems are reduced or eliminated according to the present invention by means of a connection arrangement which is characterized in that a bacteria filter is arranged within the Y-piece and is supported by radially extending support webs constituting a partition wall in the direction towards the patient up to at least the filter, said wall being arranged to separate the exhaled gas from the inhaled gas, and in that a heat and moisture exchange system is arranged in the patient attachment piece with the ability to take up heat and moisture from exhaled gas and subsequently deliver this to the inhaled gas.

In order to reduce the breathing resistance, the through-flow area of the Y-piece can hereby be enlarged in comparison with that of the patient attachment piece. By reducing the number of parts included in the connection arrangement, choking is also reduced, since otherwise this easily occurs with the connection of the various components.

A simple construction with a view to manufacturing is achieved, if the Y-piece is formed from two bowl-shaped parts and the bacteria filter is clamped between these parts substantially perpendicular to the through-flow direction.

The Y-piece of the connection arrangement normally includes three attachment nipples, namely one for the patient attachment piece, one for the inhalation tube and one for the exhalation tube. From a manufacturing point of view a particularly suitable construction is obtained when these nipples are arranged substantially parallel to each other.

Preferably the Y-piece's through-flow area is maximized in proportion to its volume through a substantially circular form, whilst its length in the flow direction is restricted to that which the function allows.

In an embodiment of the invention the connection arrangement is provided with a sample withdrawal outlet for gas samples in the flow direction for exhalation after the bacteria filter. In this way considerably dryer gas samples are obtained without secretion from the patient. The proximal pressure of the patient can also be measured via this outlet.

The patient attachment piece is preferably flexible and suitably contains a heat and moisture exchange system in the form of a wad, or similar, of a flexible material, such as fibres, with the ability to take up heat and moisture from exhaled gas and subsequently deliver this to the inhaled gas. The way that this is achieved is described in more detail in the above-mentioned US-A-4 516 573.

The sample withdrawal outlet is suitably arranged in the form of a nipple substantially in the middle of a Y-piece with, in a direction perpendicular to the through-flow direction, a somewhat drawn out through-flow area, for example substantially oval or rounded-rectangular shaped, between the attachment nipples for the inhalation resp exhalation tubes and preferably parallel to these nipples. Alternatively the sample withdrawal outlet can have the form of a nipple which is angled in respect to the other nipples. Important in both cases is, however, that it is shieldably arranged between the attachment nipples for the inhalation resp exhalation tubes.

The Y-piece requires a certain minimum volume. By way of example the included attachment nipples should have a certain standard dimension. In order that this volume affects the withdrawal sample as little as possible, the sample withdrawal outlet is suitably connected to a first sample withdrawal tube which extends from the outlet to the bacteria filter. This sample withdrawal tube can, via the bacteria filter, be connected to a second sample withdrawal tube which extends from the filter towards the patient attachment piece and preferably up to and, possibly, into this piece. In this way the effect of the Y-piece's inner volume on the withdrawal sample is substantially eliminated.

The sample withdrawal is further simplified when said first and second sample withdrawal tubes are arranged on either side of the bacteria filter and are in pressured contact therewith via a at right angles to the flow direction widened portion, for example a cone-shaped and/or cylindrical funnel-like portion. In this way the simplification of, amongst other things, the flow of the sample through the bacteria filter is achieved, whilst leakage is prevented between the inner sample withdrawal tube and the atmosphere surrounding it.

To secure the position of, and the contact between, said sample withdrawal tubes, one or both bowl-shaped halves of the Y-piece can be provided with support webs for one or both tubes.

The heat and moisture exchanger is equipped with a wad, or similar, of the above-mentioned type, which suitably could be impregnated with an antibacteria agent, for example chlorhexidine or hydrogen peroxide, and/or with a hygroscopic substance, such as magnesium chloride, lithium chloride or calcium chloride.

In an embodiment of the connection arrangement according to the invention, the above-mentioned wad or similar consists of fibres of a plastic material having a certain melting point, such as polypropylene, which are coated with another plastic material having a lower melting point, such as polyethylene, with the aid of which the fibres are bounded by heating to said lower melting point. In this way the breaking off of material from the fibre wad and its transmittal in to the patient's respiratory organs is effectively prevented.

Preferably at least one part of the patient attachment piece is made from transparent material. This part, which preferably is located nearest the patient, is left free from other material in order to serve as a secretion trap and/or inspection zone.

Said sample withdrawal outlet is appropriately arranged in a dome, or similar, directed towards the interior of the Y-piece and which is arranged to stabilize the outlet whilst also reducing the inner volume of the Y-piece.

Preferably both bowl-shaped parts of the Y-piece are provided with internal stiffening webs which are so arranged that they disturb the through-flow as little as possible, for exemple by being radially directed in relation to the principal through-flow direction, whilst also being arranged to support the bacteria filter on both sides.

Due to the fact that the present invention, according to the applicant, is closely related to the invention according to the co-pending European patent application No. EP 94102959.7 (divisional of the present application), both related to the same product, the product in its entirety is described in the following, inclusive modifications. In such a way both inventions can be easier understood.

Herebelow, the connection arrangement of the invention is described with reference to the attached drawings.

Figure 1 shows a section through a Y-piece of the connection arrangement according to the invention.

Figure 2 shows the same Y-piece as in Figure 1, but from above.

Figure 3 shows a somewhat modified Y-piece.

Figure 4 shows the last-mentioned Y-piece seen from underneath.

Figure 5 shows schematically a somewhat more complete connection arrangement according to the invention.

Figure 6 shows a plan view of the connection arrangement according to Figure 5.

Figure 7 shows a section through a nipple and a side view of another nipple belonging to the Y-piece which is included in the connection arrangement according to Figure 5.

Figure 8 shows a section along line VIII-VIII in Figure 5, but without the filter.

Figures 9-12 show views corresponding to Figures 5-8 of a modified embodiment of the connection arrangement according to the invention.

Figure 13 shows the connection arrangement according to the invention. At the same time Figure 13a shows, on a larger scale, a part of Figure 13.

Figure 14 shows the connection arrangement according to Figure 13 seen from beneath and Figure 15 shows the same seen from above.

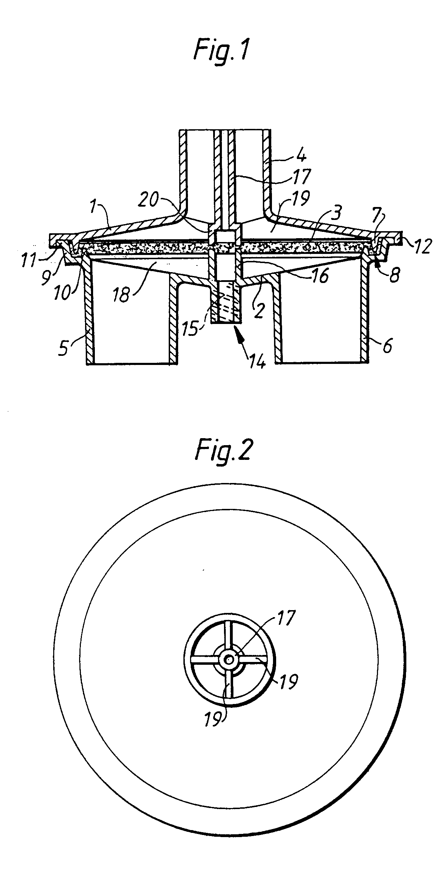

Figures 1 and 2 show a section and an end view, respectively, of a Y-piece which is intended to be included in the connection arrangement according to the invention.

This Y-piece comprises two bowl-shaped parts 1 and 2 with a bacteria filter 3 clamped therebetween. The upper bowl-shaped part 1 is provided with a nipple 4 which is intended to be connected to a patient attachment piece. The lower bowl-shaped part 2 is provided with two parallel nipples 5 and 6 which are intended to be connected to an inhalation tube resp an exhalation tube, which in turn are intended to be connected to a respirator, anaesthesia machine or similar.

The bacteria filter 3 is clamped between a circular ridge 7 on the upper bowl-shaped part 3 and a circular groove 8 in the lower bowl-shaped part 2. This groove 8 is formed from an outer flange 9 and an inner ridge 10. The flange 9 is terminated at its outer portion by a radially extending partial flange 11 which is intended to be fixed to the upper bowl-shaped part 1 by, for example, gluing or welding, preferably ultrasonic welding. The concentricity of the two bowl-shaped parts 1 and 2 is hereby facilitated by an outer peripheral flange 12 on the upper bowl-shaped part 1.

The lower bowl-shaped part 2 further comprises a sample withdrawal outlet in the form of a nipple 14, which is preferably provided with an inner or outer screw thread or other fixing means 15. The nipple 14 is connected to a first sample withdrawal tube 16 which in turn, via filter 3, is connected to a second sample withdrawal tube 17. Both the sample withdrawal tubes and the filter 3 are supported by radially extending support webs 18 resp 19. The sample withdrawal tube 17 is terminated inside with a widened cylindrical portion 20 which via the filter presses against a corresponding portion of the sample withdrawal tube 16. As is evident from Figure 2, the sample withdrawal tube 17 is located between four radially directed support webs 19.

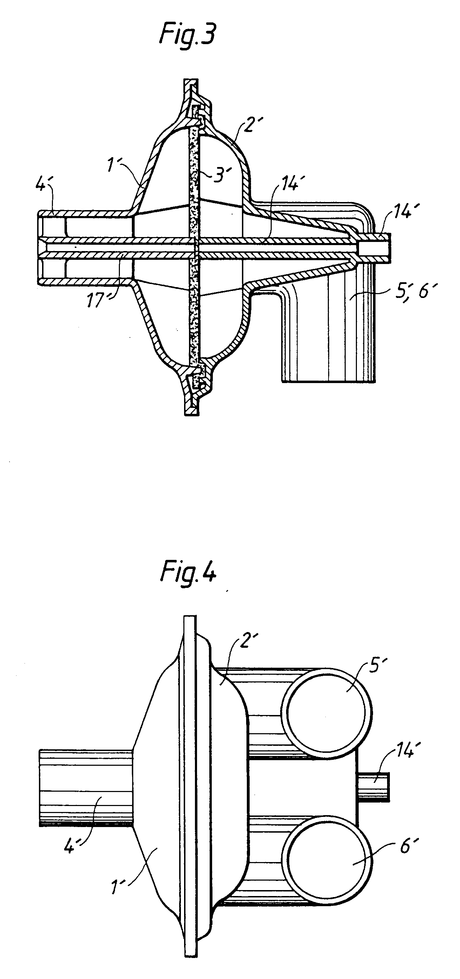

In Figures 3 and 4 there is shown the Y-piece in the connection arrangement according to the invention. The construction corresponds in principle with that according to Figure 1 and 2. Thus the same reference numerals have been used but with the addition of a dash. The most significant difference is that the attachment nipples 5' resp 6' are angularly formed. Furthermore, the sample withdrawal tubes 14' and 17' have been given a somewhat different shape.

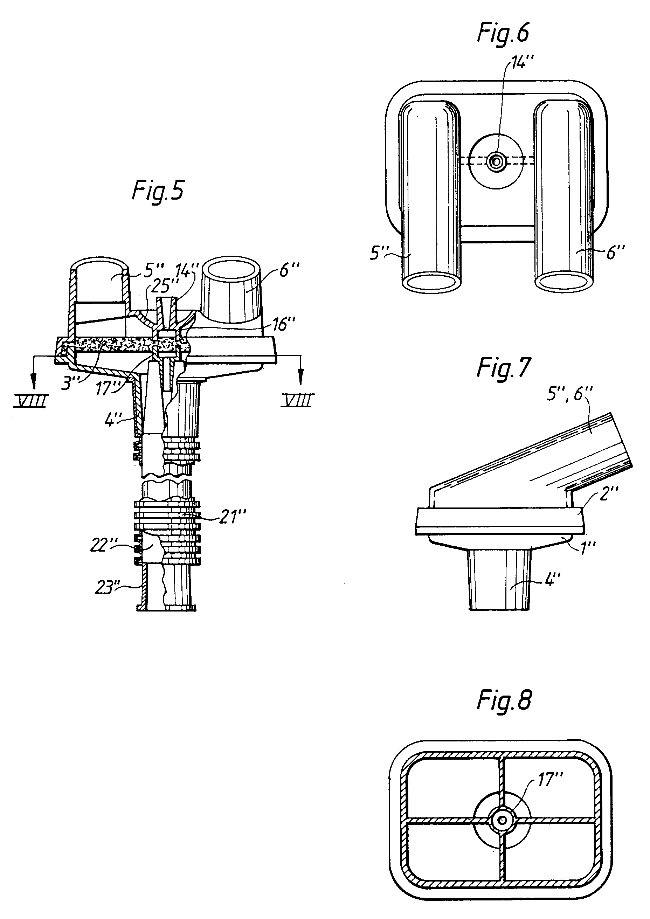

In Figures 5-8 there is shown a somewhat more complete realization of an embodiment of the connection arrangement according to the invention. This also principally corresponds with that which has been described above. Thus the same figure reference numerals are used for corresponding details, but with the addition of a double dash. Reference numerals 5'' resp 6'' thus denotes two nipples which are intended to be attached to an inhalation tube resp an exhalation tube. The bacteria filter itself is denoted by 3''. Reference numeral 14'' denotes a sample withdrawal nipple which is connected to a first sample withdrawal tube 16''. The latter is in turn via the filter 3'' in contact with a second sample withdrawal tube 17''. Reference numeral 4'' denotes a nipple with the aid of which the Y-piece is connected to a patient attachment piece 21''. This is preferably designed substantially in accordance with that described in US-A-4 516 573. It is, however, preferably of uniform thickness. Within the patient attachment piece there is preferably a wad or similar 22'' of a moisture and heat absorbing material which serves to take up heat and moisture from the exhaled gas and pass this to the inhaled gas. Finally, in Figure 5, reference numeral 23'' denotes an attachment pipe or cone with the help of which the patient attachment piece 21'' can be connected to a tracheal tube or similar.

The Y-piece shown in Figures 5-8 also consists of two bowl-shaped parts 1'' resp 2'', but differs from the above described Y-pieces in that, for example, these parts have been given a rounded rectangular shape. Furthermore, the attachment nipples 5'' and 6'' are arranged at a different angle to that which is shown in, for example, Figures 3 and 4. Finally, reference numerals 25'' denotes a dome directed towards the interior of the Y-piece in which the sample withdrawal nipple 14'' is arranged. Thanks to this arrangement the Y-piece's inner volume is reduced whilst the sample withdrawal tube 16'' is stabilized.

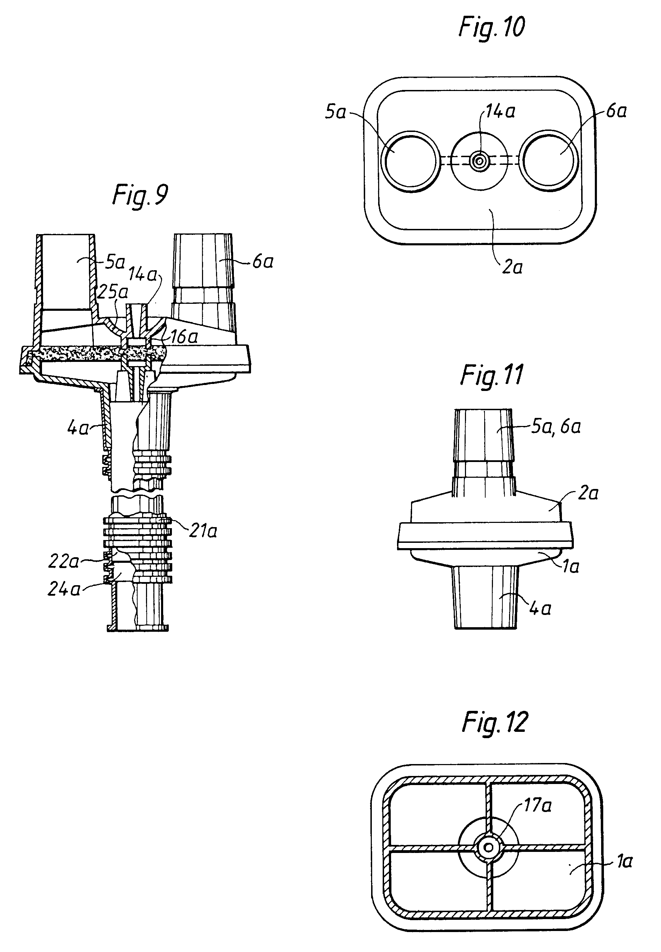

The construction according to Figures 9-12 substantially corresponds with that according to Figures 5-8. Corresponding details have thus been given the same figure reference numerals, but with the suffix a instead of the double dashes used in Figures 5-8. The construction according to Figures 9-12 differs from that according to Figures 5-8 in that the nipples 5a and 6a are arranged parallel to the nipple 4a. Furthermore, the fibre wad 22a does not fill the whole patient attachment piece 21a. A free space 24a is left nearest the patient, which is intended to serve as a secretion trap and which can also serve as an inspection zone if the patient attachment piece is made from a transparent material. Finally, reference numeral 25a denotes a dome directed towards the interior of the Y-piece in which the sample withdrawal nipple 14a is arranged. Thanks to this arrangement the Y-piece's inner volume is reduced whilst the sample withdrawal tube 16a is stabilized.

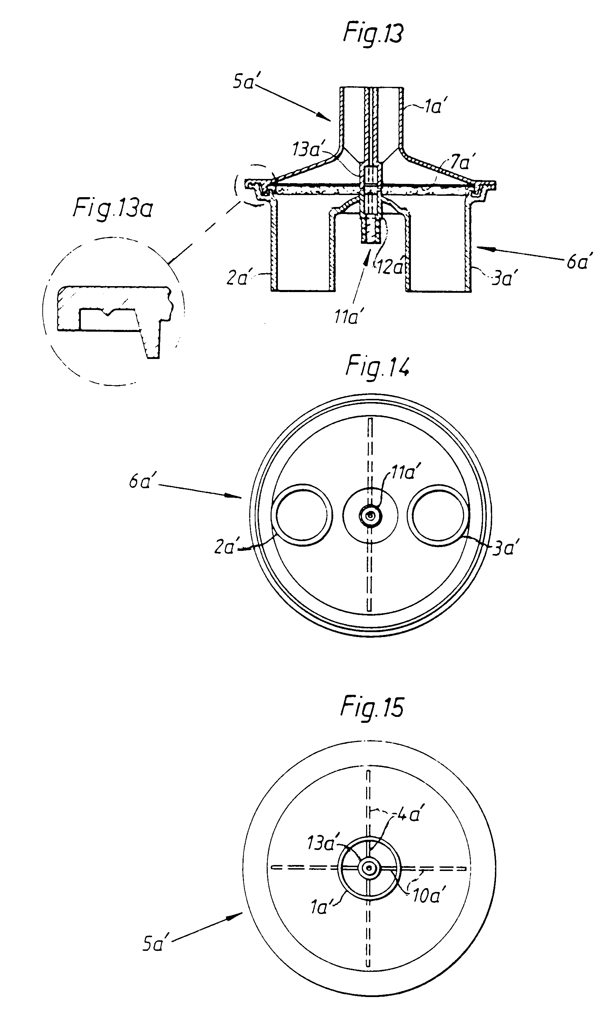

Figures 13-15 are showing the Y-piece made in accordance with the invention. The embodiment corresponds essentially to the one shown in Figures 1 and 2. Different reference numerals have, however, been used, but all with the addition of a'. The Y-piece described comprises two bowl-shaped parts 5a' and 6a' with a bacteria filter 7a' clamped therebetween. The upper bowl-shaped part 5a' is provided with a nipple 1a', which is intended to be connected to a patient attachment piece. The lower bowl-shaped part 6a' is provided with two parallel nipples 2a' and 3a' which are intended to be connected to an inhalation tube resp exhalation tube, which in turn are intended to be connected to a respirator, anaesthesia machine or similar.

The bacteria filter 7a' is clamped in the same way as the filter 3 in Figure 1. The lower bowl-shaped part 6a' further comprises a sample withdrawal outlet in the form of a nipple 11a'. The nipple 11a' is connected to a first sample withdrawal tube 12a' which in turn, via the filter 7a', is connected to a second sample withdrawal tube 13a'.

The main difference between Figures 1-2 and Figures 13-15 is that the supporting webs constituting the partition wall, which is defined in the characterizing portion of the present invention, is clearly marked out with the reference numeral 4a'. This partition wall 4a' is preferably made in one piece with the two sample withdrawal tubes 12a' and 13a' and separates exhaled gas from inhaled gas. As shown in Figure 15 the part 5a' may also be provided with a second partition wall 10a' intended to support the filter 7a'. In the embodiment of Figures 1-12, the partition wall is not shown for clarity reasons.

## Claims

1. Connection arrangement for connecting a patient to a respirator, anaestesia machine or similar, comprising a patient attachment piece (21'', 21a), a so-called Y-piece (1, 2) disconnectable from said patient attachment piece and arranged to join said patient attachment piece to an inhalation tube and an exhalation tube, respectively, a bacteria filter (3) and a heat and moisture exchange system (22'', 22a) , **characterized** in that said bacteria filter is arranged within said Y-piece (1, 2) and is supported by radially extending support webs (18, 19) constituting a partition wall (4a') in the direction towards the patient up to at least the filter (3), said wall being arranged to separate the exhaled gas from the inhaled gas, and in that said heat and moisture exchange system is arranged in said patient attachment piece (21'', 21a) with the ability to take up heat and moisture from exhaled gas and subsequently deliver this to the inhaled gas.

2. Connection arrangement according to claim 1, **characterized** in that the through-flow area of the Y-piece (1, 2) is enlarged in comparison with that of the patient attachment piece.

3. Connection arrangement according to claim 1 or 2, **characterized** in that the Y-piece (1, 2) is formed from two bowl-shaped parts (1, 2) wherein the bacteria filter (3) is clamped between these parts substantially perpendicular to the through-flow direction.

4. Connection arrangement according to anyone of the preceding claims wherein the Y-piece (1, 2) comprises three attachment nipples, namely one (4) for the patient attachment piece, one (5) for the inhalation tube and one (6) for the exhalation tube, **characterized** in that said nipples (4-6) are arranged substantially parallel to each other.

5. Connection arrangement according to anyone of the preceding claims, **characterized** in that the Y-piece's (1, 2) through-flow area is maximized in proportion to its volume by a substantially circular form, whilst its length in the flow direction is restricted to that which the function allows.

6. Connection arrangement according to anyone of the preceding claims, **characterized** in that it is provided with a sample withdrawal outlet (14) for gas samples in the flow direction for exhalation after the bacteria filter.

7. Connection arrangement according to anyone of the preceding claims, **characterized** in that the patient attachment piece (21'' or 21a) is flexible and in that said heat and moisture exchange system is in the form of a wad or similar (22'' or 22a) of a flexible material, such as fibres.

8. Connection arrangement according to claim 4 and 6, **characterized** in that the sample withdrawal outlet (14'' or 14a) is arranged, preferably in a form of a nipple, substantially in the middle between the attachment nipples for the inhalation resp. exhalation tubes and preferably parallel to these tubes whereby the sample withdrawal outlet has in a direction perpendicular to its through-flow direction a somewhat drawn-out through-flow area, for example substantially oval or rounded-rectangular shaped, between the attachment nipples (5'', 6''; 5a, 6a) for the inhalation resp. exhalation tubes and preferably parallel to these nipples.

9. Connection arrangement according to claim 6, **characterized** in that the sample withdrawal outlet (14) is connected to a first sample withdrawal tube (16) which extends from the bacteria filter (3) to the outlet.

10. Connection arrangement according to claim 9, **characterized** in that said first sample withdrawal tube (16) via the bacteria filter (3) is connected to a second sample withdrawal tube (17) which extends from the filter towards the patient attachment piece and preferably up to and, possibly, into this piece.

11. Connection arrangement according to claim 10, **characterized** in that said first and second sample withdrawal tubes (16, 17) are arranged on either side of the bacteria filter and are in pressurized contact therewith via a widened portion at right angles to the flow direction, for example a cone-shaped and/or cylindrical funnel-like portion (e.g. 20).

12. Connection arrangement according to claims 3 and 10, **characterized** in that one or both bowl-shaped halves of the Y-piece (1, 2) are provided with support webs (18, 19) for said first and/or second sample withdrawal tube.

13. Connection arrangement according to claim 7, **characterized** in that said wad or similar (22'', 22a) is impregnated with an antibacteria agent, for example chlorhexidine or hydrogen peroxide, and/or with a hygroscopic substance, such as magnesium chloride, lithium chloride or calcium chloride.

14. Connection arrangement according to claim 7, **characterized** in that said wad or similar (22'', 22a) consists of fibres of a plastic material having a certain melting point, such as polyprophylene, which are coated with another plastic material having a lower melting point, such as polyethylene, with the aid of which the fibres are bonded by heating to said lower melting point.

15. Connection arrangement according to any one of the preceding claims, **characterized** in that at least one part (24a) of the patient attachment piece (21a) is transparent and no other material is in this part, preferably nearest the patient, so that it can serve as a secretion trap and/or inspection zone.

16. Connection arrangement according to any one of the claims 6-12, **characterized** in that the sample withdrawal outlet (14'', 14a) is arranged in a dome, or similar, directed towards the interior of the Y-piece (1, 2) and which is arranged to stabilize the outlet whilst also reducing the inner volume of the Y-piece.

17. Connection arrangement according to any one of the claims 12-16, **characterized** in that said internal stiffening webs (18, 19) are so arranged in both bowl-shaped parts of the Y-piece (1, 2) that they disturb the through-flow as little as possible by being radially directed in relation to the principal through-flow direction, whilst also being arranged to support the bacteria filter (3) on both sides.

## Patentansprüche

1. Verbindungsvorrichtung zum Verbinden eines Patienten mit einem Beatmungsgerät, Narkosegerät oder dgl., umfassend ein Patienten-Festlegungsstück (21'', 21a), ein sogenanntes Y-Stück (1, 2), welches von dem Patienten-Festlegungsstück abtrennbar ist und so angeordnet ist, um das Patienten-Festlegungsstück mit einem Inhalationsschlauch bzw. einem Exhalationsschlauch zu verbinden, ein Bakterienfilter (3) und ein Wärme- und Feuchtigkeitsaustauschsystem (22'', 22a), dadurch gekennzeichnet, daß das Bakterienfilter in dem Y-Stück (1, 2) angeordnet ist und durch sich radial erstreckende Stutzstege (18, 19) getragen ist, welche in Richtung zu dem Patienten bis zu wenigstens dem Filter (3) eine Trennwand (4a') bilden, welche Wand so angeordnet ist, um das ausgeatmete Gas von dem eingeatmeten Gas zu trennen, und daß das Wärme- und Feuchtigkeisaustauschsystem in dem Patienten-Festlegungsstück (21'', 21a) angeordnet ist, mit der Fähigkeit, Wärme und Feuchtigkeit von dem ausgeatmeten Gas aufzunehmen und anschließend diese in das eingeatmete Gas abzugeben.

2. Verbindungsvorrichtung nach Anspruch 1, dadurch gekennzeichnet, daß der Durchflußbereich des Y-Stückes (1, 2) im Vergleich zu dem Patienten-Festlegungsstück vergrößert ist.

3. Verbindungsvorrichtung nach Anspruch 1 oder 2, dadurch gekennzeichnet, daß das Y-Stück (1, 2) aus zwei schalenfömigen Teilen (1, 2) ausgebildet ist, wobei das Bakterienfilter (3) zwischen diesen Teilen im wesentlichen normal auf die Durchflußrichtung festgeklemmt ist.

4. Verbindungsvorrichtung nach einem der vorhergehenden Ansprüche, worin das Y-Stück (1, 2) drei Anlenknippel, nämlich einen (4) für das Patienten-Festlegungsstück, einen (5) für den Inhalationsschlauch und einen (6) für den Exhalationsschlauch, aufweist, dadurch gekennzeichnet, daß diese Nippel (4 - 6) im wesentlichen parallel zueinander angeordnet sind.

5. Verbindungsvorrichtung nach einem der vorhergehenden Ansprüche, dadurch gekennzeichnet, daß die Durchflußfläche durch das Y-Stück (1, 2) im Verhältnis zu seinem Volumen durch eine im wesentlichen kreisförmige Form maximiert ist, wohingegen seine Länge in der Flußrichtung soweit beschränkt ist, wie dies die Funktionsfähigkeit ermöglicht.

6. Verbindungsvorrichtung nach einem der vorhergehenden Ansprüche, dadurch gekennzeichnet, daß sie mit einem Probennahmeauslaß (14) für Gasproben in der Exhalations-Flußrichtung nach dem Bakterienfilter versehen ist.

7. Verbindungsvorrichtung nach einem der vorhergehenden Ansprüche, dadurch gekennzeichnet, daß das Patienten-Festlegungsstück (21'' oder 21a) flexibel ist und daß das Wärme- und Feuchtigkeitsaustauschsystem in Form einer Wattierung oder dgl. (22'' oder 22a) aus einem flexiblen Material, wie etwa Fasern, gebildet ist.

8. Verbindungsvorrichtung nach Anspruch 4 und 6, dadurch gekennzeichnet, daß der Probennahmeauslaß (14'' oder 14a) vorzugsweise in Form eines Nippels im wesentlichen in der Mitte zwischen den Anlenknippeln für die Inhalations- bzw. Exhalationsschläuche und vorzugsweise parallel zu diesen Schläuchen angeordnet ist, wobei der Probennahmeauslaß in einer Richtung normal zu seiner Durchflußrichtung einen etwas ausgezogenen Durchflußbereich, beispielsweise im wesentlichen oval oder abgerundet rechteckig geformt, zwischen den Anlenknippeln (5'', 6''; 5a, 6a) für die Inhalations- bzw. Exhalationsschläuche und vorzugsweise parallel zu diesen Nippeln angeordnet aufweist.

9. Verbindungsvorrichtung nach Anspruch 6, dadurch gekennzeichnet, daß der Probennahmeauslaß (14) mit einem ersten Probennahmeschlauch (16) verbunden ist, welcher sich von dem Bakterienfilter (3) zu dem Auslaß erstreckt.

10. Verbindungsvorrichtung nach Anspruch 9, dadurch gekennzeichnet, daß der erste Probennahmeschlauch (16) über das Bakterienfilter (3) mit einem zweiten Probennahmeschlauch (17) verbunden ist, welcher sich von dem Filter in Richtung zu dem Patienten-Festlegungsstück und vorzugsweise bis zu diesem und gegebenenfalls in dieses Stück hinein erstreckt.

11. Verbindungsvorrichtung nach Anspruch 10, dadurch gekennzeichnet, daß die ersten und zweiten Probennahmeschläuche (16, 17) auf den beiden Seiten des Bakterienfilters angeordnet sind und damit über einen ausgeweiteten Bereich in rechten Winkeln zu der Flußrichtung, beispielsweise einem konisch geformten und/oder zylindrisch-trichterförmigen Bereich (z.B. 20), in Druckkontakt stehen.

12. Verbindungsvorrichtung nach Anspruch 3 und 10, dadurch gekennzeichnet, daß eine oder beide schalenförmig geformten Hälften des Y-Stückes (1, 2) mit Stützstegen (18, 19) für die ersten und/oder zweiten Probennahmeschläuche versehen ist.

13. Verbindungsvorrichtung nach Anspruch 7, dadurch gekennzeichnet, daß die Wattierung oder dgl. (22'', 22a) mit einem antibakteriellen Agens, beispielsweise Chlorhexidin oder Wasserstoffperoxid, und/oder einer hygroskopischen Substanz, wie Magnesiumchlorid, Lithiumchlorid oder Calziumchlorid, imprägniert ist.

14. Verbindungsvorrichtung nach Anspruch 7, dadurch gekennzeichnet, daß die Wattierung oder dgl. (22'', 22a) aus Fasern aus einem Kunststoffmaterial besteht, welches einen bestimmten Schmelzpunkt aufweist, wie Polypropylen, welche mit einem anderen Kunststoffmaterial, welches einen niedrigeren Schmelzpunkt aufweist, wie Polyethylen, beschichtet sind, mit deren Hilfe die Fasern durch Erhitzen auf den niedrigeren Schmelzpunkt verbunden sind.

15. Verbindungsvorrichtung nach einem der vorhergehenden Ansprüche, dadurch gekennzeichnet, daß wenigstens ein Teil (24a) des Patienten-Festlegungsstückes (21a) transparent ist und sich in diesem Bereich, vorzugsweise nächst dem Patienten, kein anderes Material befindet, so daß es als eine Sekretionsfalle und/oder als Beobachtungsbereich dienen kann.

16. Verbindungsvorrichtung nach einem der Ansprüche 6 bis 12, dadurch gekennzeichnet, daß der Probennahmeauslaß (14'', 14a) in einer Kuppel oder dgl. angeordnet ist, welche in Richtung zum Inneren des Y-Stückes (1, 2) gerichtet ist, und welches so angeordnet ist, um den Auslaß zu stabilisieren, während auch das Innenvolumen des Y-Stückes reduziert ist.

17. Verbindungsvorrichtung nach einem der Ansprüche 12 bis 16, dadurch gekennzeichnet, daß die inneren Versteifungsstege (18, 19) so in den beiden schalenförmigen Teilen des Y-Stückes (1, 2) angeordnet sind, daß sie den Durchfluß so wenig wie möglich stören, indem sie radial in bezug auf die Hauptdurchflußrichtung gerichtet sind, während sie auch so angeordnet sind, um das Bakterienfilter (3) auf beiden Seiten abzustützen.

## Revendications

1. Dispositif de raccordement servant à raccorder un patient à un respirateur, un appareil d'anesthésie ou analogue, comprenant un élément (21'',21a) de raccordement à un patient, une pièce dite pièce en Y (1,2), qui peut être déconnectée de l'élément de raccordement au patient et est agencée de manière à réunir ledit élément de raccordement au patient respectivement à un tube d'aspiration et à un tube d'expiration, un filtre à bactéries (3) et un système d'échange de chaleur et d'humidité (22'',22a), caractérisé en ce que ledit filtre bactérien est disposé dans ladite pièce en Y (1,2) et est supporté par des âmes radiales de support (18,19) constituant une cloison de séparation (4a') s'étendant en direction du patient au moins jusqu'au filtre (3), ladite paroi étant agencée de manière à séparer le gaz expiré du gaz aspiré, et en ce que ledit système d'échange de chaleur et d'humidité est disposé dans ledit élément (21'',21a) de raccordement au patient, de manière à pouvoir absorber la chaleur et l'humidité des gaz expirés et de les envoyer ensuite aux gaz aspirés.

2. Dispositif de raccordement selon la revendication 1, caractérisé en ce que la surface de traversée de la pièce en Y (1,2) est élargie par rapport à celle de l'élément de raccordement au patient.

3. Dispositif de raccordement selon la revendication 1 ou 2, caractérisé en ce que la pièce en Y (1,2) est formée de deux parties en forme de bols (1,2), le filtre à bactéries (3) étant serré entre ces parties, sensiblement perpendiculairement à la direction de traversée.

4. Dispositif de raccordement selon l'une quelconque des revendications précédentes, dans lequel la pièce en Y (1,2) comprend trois raccords filetés de fixation, à savoir l'un (4) pour l'élément de raccordement au patient, l'un (5) pour le tube d'aspiration et l'un (6) pour le tube d'expiration, caractérisé en ce que lesdits raccords filetés (4-6) sont disposés de manière à être essentiellement parallèles entre eux.

5. Dispositif de raccordement selon l'une quelconque des revendications précédentes, caractérisé en ce que la surface de traversée des pièces en Y (1,2) est rendue maximale proportionnellement à son volume, au moyen d'une forme essentiellement circulaire, tandis que sa longueur dans la direction d'écoulement est limitée à celle que permet le fonctionnement.

6. Dispositif de raccordement selon l'une quelconque des revendications précédentes, caractérisé en ce qu'il est prévu une sortie (14) de prélèvement d'échantillon de gaz dans la direction d'écoulement pour l'expiration, en aval du filtre à bactéries.

7. Dispositif de raccordement selon l'une quelconque des revendications précédentes, caractérisé en ce que l'élément (21'' ou 21a) de raccordement du patient est flexible et en ce que ledit système d'échange de chaleur et d'humidité se présente sous la forme d'un tampon ou analogue (22'' ou 22a) formé d'un matériau flexible, par exemple des fibres.

8. Dispositif de raccordement selon les revendications 4 et 6, caractérisé en ce que la sortie (14'', 14a) de prélèvement d'échantillons est disposée de préférence sous la forme d'un raccord fileté, essentiellement au milieu entre les raccords filetés de fixation pour respectivement le tube d'aspiration et le tube d'expiration, et de préférence parallèlement à ces tubes, la sortie de prélèvement d'échantillons possédant dans une direction perpendiculaire à sa direction de traversée, une surface de traversée légèrement étirée, par exemple de forme sensiblement ovale ou rectangulaire ou arrondie, entre les raccords filetés de fixation (5'',6'';5a;6a) respectivement pour les tubes d'inspiration et d'expiration, et de préférence parallèlement à ces raccords filetés.

9. Dispositif de raccordement selon la revendication 6, caractérisé en ce que la sortie (14) de prélèvement d'échantillons est raccordée à un premier tube (16) de prélèvement d'échantillons, qui s'étend depuis le filtre à bactéries (3) en direction de la sortie.

10. Dispositif de raccordement selon la revendication 9, caractérisé en ce que ledit premier tube (16) de prélèvement d'échantillons est raccordé par l'intermédiaire du filtre à bactéries (3) par un second tube (17) de prélèvement d'échantillons, qui s'étend depuis le filtre en direction de l'élément de raccordement au patient et de préférence jusqu'à et éventuellement jusque dans cet échantillon.

11. Dispositif de raccordement selon la revendication 10, caractérisé en ce que lesdits premier et second tubes (16,17) de prélèvement d'échantillons sont disposés des deux côtés du filtre à bactéries et sont placés en contact sous pression avec ce dernier par l'intermédiaire d'une partie élargie, perpendiculairement à la direction d'écoulement, par exemple une partie en forme de cône et/ou en forme d'entonnoir cylindrique (par exemple 20).

12. Dispositif de raccordement selon les revendications 3 et 10, caractérisé en ce qu'une moitié ou les deux moitiés en forme de bols de la pièce en Y (1,2) comportent des âmes de support (18,19) pour ledit premier et/ou ledit second tube de prélèvement d'échantillons.

13. Dispositif de raccordement selon la revendication 7, caractérisé en ce que ledit tampon ou analogue (22'',22a) est imprégné d'un agent antibactérien par exemple de la chlorhexidine ou du peroxyde d'hydrogène et/ou avec une substance hydroscopique, tel que du chlorure de magnésium, du chlorure de lithium ou du chlorure de calcium.

14. Dispositif de raccordement selon la revendication 7, caractérisé en ce que ledit tampon ou analogue (22'',22a) est constitué de fibres de matière plastique possédant un certain point de fusion, tel que du polyprophylène, qui sont recouvertes d'une autre matière plastique possédant un point de fusion inférieur, tel que du polyéthylène, à l'aide duquel les fibres sont réunies par chauffage audit point de fusion inférieur.

15. Dispositif de raccordement selon l'une quelconque des revendications précédentes, caractérisé en ce qu'au moins une partie (24a) de l'élément (21a) de raccordement au patient est transparent et qu'aucun autre matériau n'est présent dans cette partie, de préférence la plus proche du patient, de sorte que cette partie peut servir de réceptacle à secrétion et/ou de zone d'inspection.

16. Dispositif de raccordement selon l'une quelconque des revendications 6-12, caractérisé en ce que la sortie (14'',14a) de prélèvement d'échantillons est disposée dans un dôme ou analogue, dirigé vers l'intérieur de la pièce en Y (1,2) et qui est agencé de manière à stabiliser la sortie, tout en réduisant le volume intérieur de la pièce en Y.

17. Dispositif de raccordement selon l'une quelconque des revendications 12-16, caractérisé en ce que lesdites âmes internes de rigidification (18,19) sont disposées dans les deux parties en forme de bols de la pièce en Y (1,2) de telle sorte qu'elles perturbent aussi peu que possible l'écoulement traversant, en étant dirigées radialement par rapport à la direction de traversée principale, tout en étant également agencées de manière à supporter sur ses deux côtés, le filtre à bactéries (3).
